# EUROPÄISCHE PATENTSCHRIFT

(11) **EP 3 826 554 B1**
(45) Veröffentlichungstag und Bekanntmachung des Hinweises auf die Patenterteilung: **11.09.2024**
(21) Anmeldenummer: 18750094.7
(22) Anmeldetag: 25.07.2018
(51) Int. Cl.: A61B 17/14

(54) **CHIRURGISCHE SÄGEVORRICHTUNG**
SURGICAL SAW APPARATUS
DISPOSITIF DE SCIE CHIRURGICALE

(43) Veröffentlichungstag der Anmeldung: 02.06.2021
(73) Patentinhaber: Studer, Armin, 6330 Cham (CH)
(72) Erfinder: JANSEN-TROY, Arne, 78333 Stockach (DE); STUDER, Armin, 6330 Cham (CH)
(74) Vertreter: Daub, Thomas
(86) Internationale Anmeldenummer: PCT/CH2018/000032
(87) Internationale Veröffentlichungsnummer: WO 2020/019086

(56) Entgegenhaltungen:
- WO-A1-2011/162736
- DE-A1- 102009 048 835
- DE-U1- 8 706 162
- US-A- 3 978 862

## Beschreibung

Die Erfindung bezieht sich auf eine chirurgische Sägevorrichtung gemäss dem Oberbegriff des Patentanspruchs 1.

Eine chirurgische Sägevorrichtung, welche ein Getriebe zur Erzeugung einer Kreisbewegung des schneidenden Elements, insbesondere eines Sägeblatts umfasst, ist aus den Dokumenten DE 10 2009 048 835 A1 und DE 87 06 162 U1 bekannt. Durch die reine Kreisbewegung des Sägeblatts in einer Ebene wird ein optimaler Weichgewebeschutz erreicht. Das Getriebe zur Erzeugung der Kreisbewegung des Sägeblatts umfasst einen Pleuel, welcher durch zwei synchron den Pleuel antreibende Exzenter angetrieben wird. Dabei befindet sich je ein antreibender Exzenter in einem vorderen Abschnitt und in einem hinteren Abschnitt des Pleuels. Die beiden antreibenden Exzenter sind mechanisch miteinander gekoppelt, z.B. mittels eines Zahnradgetriebes oder Riementriebs. Diese bekannte Sägevorrichtung weist jedoch den Nachteil auf, dass die Vorrichtung zwei synchron angetriebene Exzenter umfasst, so dass zur Erzeugung der synchronen Rotationsbewegung der beiden Exzenter diese mechanisch gekoppelt sein müssen, was eine aufwendige Konstruktion der den Pleuel antreibenden Antriebsvorrichtung zur Folge hat.

Hier will die Erfindung Abhilfe schaffen. Der Erfindung liegt die Aufgabe zugrunde, eine chirurgische Sägevorrichtung zur Verfügung zu stellen, welche eine konstruktiv einfache Antriebsvorrichtung für einen eine Kreisbewegung ausführenden Pleuel aufweist.

Die Erfindung löst die gestellte Aufgabe mit einer chirurgischen Sägevorrichtung, welche die Merkmale des Anspruchs 1 aufweist.

Die durch die Erfindung erreichten Vorteile sind im Wesentlichen darin zu sehen, dass dank der erfindungsgemässen chirurgischen Sägevorrichtung:
- eine exakte Kreisbewegung des Pleuels und damit des am Pleuel befestigten Sägeblatts mit nur einem angetriebenen Exzenter und einer Führungseinrichtung mit einer rein translatorischen Führung für den Pleuel relativ zu zwei transversal zueinander stehenden Führungsachsen ermöglicht wird;
- nur eine angetriebenen Exzenterwelle verwendet wird, so dass sich ein Zahnradgetriebe oder ein Riementrieb erübrigt, so dass eine kleine Gesamtbaugrösse der Sägevorrichtung erreichbar ist. Aufgrund dieser kleinen Gesamtbaugrösse kann die Anwendung der Sägevorrichtung erheblich verbessert werden. Dies betrifft das Sichtfeld des Operateurs und die Bewegungsfreiheit, was insbesondere für eine geschlossene Schnittführung wichtig ist (mit Inline Säge), z.B. für Trepanationsschnitte am Schädelknochen, bei dem ein komplettes Knochenstück entnommen wird;
- weniger Bauteile notwendig sind, was weniger Hinterschnitte und überlappende Bauteile (z.B. Zähne im Eingriff) bedeutet, so dass die Sägevorrichtung einfacher und besser gereinigt werden kann und damit eine erhöhte Hygiene erreichbar ist;
- ebenso durch die geringere Anzahl Bauteile der Wartungsaufwand reduziert wird, der Verschleiss minimiert und das Gewicht reduziert werden kann, was wiederum einen erheblichen Einfluss auf die Anwendbarkeit der Sägevorrichtung hat;
- zudem eine geringere Anzahl Bauteile auch eine Risikominimierung betreffend das Versagen von Bauteilen bedeutet; und
- die Sägevorrichtung mit weniger Fertigungsaufwand hergestellt werden kann, da weniger Bauteile vorliegen und grössere Toleranzbereiche der Einzelteil möglich sind.

Der Weichgebeschutz im Allgemeinen basiert auf einer Amplitude, respektive Auslenkung des Sägeblatts, die kleiner ist als die Ausdehnung des Weichgewebes vor dem Zerreißen/Zerschneiden. Knöcherne Strukturen sind nicht in der Lage auszuweichen, wie es beim Weichgewebe der Fall ist. Das Weichgewebe ist zäh und kann entsprechend in kleiner Auslenkung mitschwingen, so dass kein Schnitt möglich ist.

Wenn die Bewegung eine elliptische oszillierende Bewegung mit einem Drehpunkt ist, ergibt sich das Problem, dass nur an einem Punkt eine ideale Kreisbewegung stattfindet. Alle Punkte in Richtung des Exzenters haben eine kleinere Abtragsrate als der ideale Punkt - alle Punkte darüber hinaus kragen immer weiter von der Mittelachse aus, sodass ab einer bestimmten Länge der Weichgewebeschutz nicht mehr gegeben ist.

Untersuchungen haben gezeigt, dass ein grosser Anteil der eingebrachten Wärmeenergie bei spanenden Verfahren in den Spänen verbleibt - dies gilt ebenfalls für das Knochenmaterial, welches zerschnitten wird. Bei einer kreisförmigen Bewegung kann eine optimale Spanabfuhr erzeugt werden, da die Späne von einem Sägezahn zum nächsten gelangen und somit aus dem Schnittspalt entfernt werden. Besonders kritisch ist die Überhitzung des Knochens, was zur Nekrose führen kann. Mit der erfindungsgemässen Sägevorrichtung kann das Risiko einer Überhitzung stark reduziert werden.

In einer speziellen Ausführungsform umfasst der Pleuel zwei Seitenflächen, welche sich parallel zu einer Ebene erstrecken, wobei die Ebene durch die Längsachse des Pleuels und durch eine Lagerachse des Lagers für den exzentrischen Abschnitt der Exzenterwelle im hinteren Abschnitt des Pleuels aufgespannt wird.

In einer weiteren Ausführungsform bilden die ersten Mittel der Führungseinrichtung eine Linearführung für die parallelen Seitenflächen des Pleuels.

Vorzugsweise weist die Linearführung an jeder der zwei Seitenflächen des Pleuels ein oder mehrere Führungselemente auf.

In einer anderen Ausführungsform umfassen die ersten Mittel ein relativ zum Gehäuse transversal bewegbares Bauteil, wobei die Führungselemente der Linearführung an oder in diesem Bauteil angeordnet sind.

Vorzugsweise bilden die zweiten Mittel relativ zur Längsachse des Pleuels eine Transversalführung für das Bauteil.

In einer weiteren Ausführungsform weist der exzentrische zweite Abschnitt gegenüber dem ersten Abschnitt der Exzenterwelle eine Exzentrizität e in einem Bereich zwischen 0,25 mm und 0,5 mm auf. Durch diese Exzentrizität e ergibt sich die effizienteste Amplitude (Durchmesser der Kreisbewegung) in einem Bereich von 0,5 bis 1 mm. Durch die Parallele Zwangslage des Pleuels ist sie auch in weiteren Positionen Kreisförmig, d.h. das Sägeblatt bewegt sich ausschließlich kreisförmig in der Ebene des Pleuels.

In einer weiteren Ausführungsform weist die Antriebsachse der Antriebseinheit eine Drehzahl von mindestens 9'000 Umdrehungen/min., vorzugsweise von mindestens 10'000 Umdrehungen/min. auf.

In wiederum einer weiteren Ausführungsform weist die Antriebsachse der Antriebseinheit eine Drehzahl von höchstens 22'000 Umdrehungen/min., vorzugsweise von höchstens 20'000 Umdrehungen/min. auf.

Die hohe Frequenz ist von Nöten um einen akzeptablen Abtrag von knöchernen Strukturen zu ermöglichen.

In einer anderen Ausführungsform umfassen die Linearführung und/oder die Transversalführung zwei oder mehr Wälzlager. Damit ist der Vorteil erreichbar, dass die Reibung zwischen der Linearführung und dem Pleuel und der Transversalführung für das bewegbare Bauteil erheblich reduziert werden kann.

In einer weiteren Ausführungsform umfasst die chirurgische Sägevorrichtung zusätzlich ein Sägeblatt.

Die Erfindung und Weiterbildungen der Erfindung werden im Folgenden anhand der teilweise schematischen Darstellungen mehrerer Ausführungsbeispiele noch näher erläutert.

Es zeigen:
Fig. 1 eine Ansicht der Umlenkeinrichtung einer ersten Ausführungsform der erfindungsgemässen chirurgischen Sägevorrichtung;
Fig. 2 einen Längsschnitt durch die in Fig. 1 dargestellte Ausführungsform der erfindungsgemässen chirurgischen Sägevorrichtung entlang der Linie I - I;
Fig. 3 einen perspektivische Darstellung eines Längsschnitts durch die Umlenkeinrichtung einer zweiten Ausführungsform der erfindungsgemässen chirurgischen Sägevorrichtung;
Fig. 4 eine Ansicht der Umlenkeinrichtung der in Fig. 3 dargestellten Ausführungsform der erfindungsgemässen chirurgischen Sägevorrichtung;
Fig. 5 einen Längsschnitt durch die in Fig. 3 dargestellte Ausführungsform der erfindungsgemässen chirurgischen Sägevorrichtung entlang der Linie II - II;
Fig. 6 eine Ansicht der Umlenkeinrichtung einer dritten Ausführungsform der erfindungsgemässen chirurgischen Sägevorrichtung;
Fig. 7 eine Teilansicht der Umlenkeinrichtung der in Fig. 6 dargestellten Ausführungsform der erfindungsgemässen chirurgischen Sägevorrichtung;
Fig. 8 eine Schnittdarstellung der in den Fig. 3 - 5 dargestellten Ausführungsform der erfindungsgemässen chirurgischen Sägevorrichtung;
Fig. 9 eine perspektivische Darstellung der in den Fig. 3 - 5 dargestellten Ausführungsform der erfindungsgemässen chirurgischen Sägevorrichtung;
Fig. 10 eine Schnittdarstellung einer weiteren Ausführungsform der erfindungsgemässen chirurgischen Sägevorrichtung mit der in den Fig. 3 - 5 dargestellten Umlenkeinrichtung; und
Fig. 11 eine perspektivische Darstellung der in Fig. 10 dargestellten Ausführungsform der erfindungsgemässen chirurgischen Sägevorrichtung.

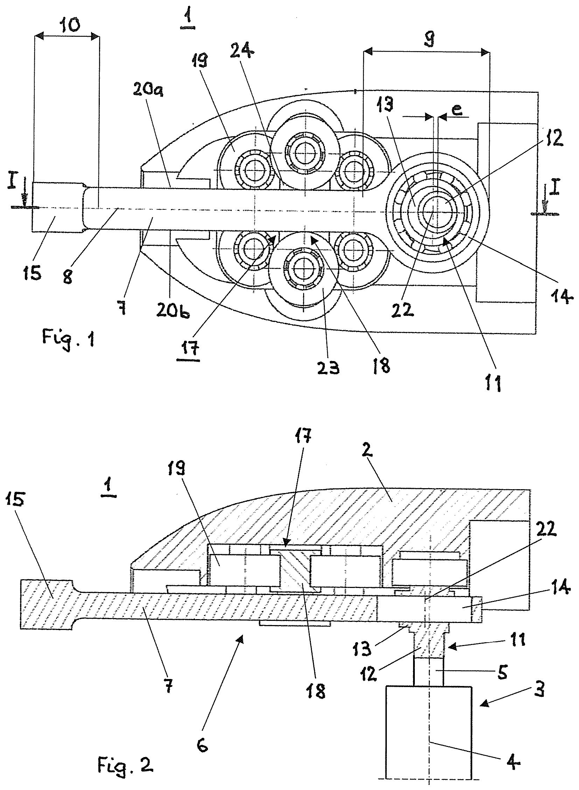

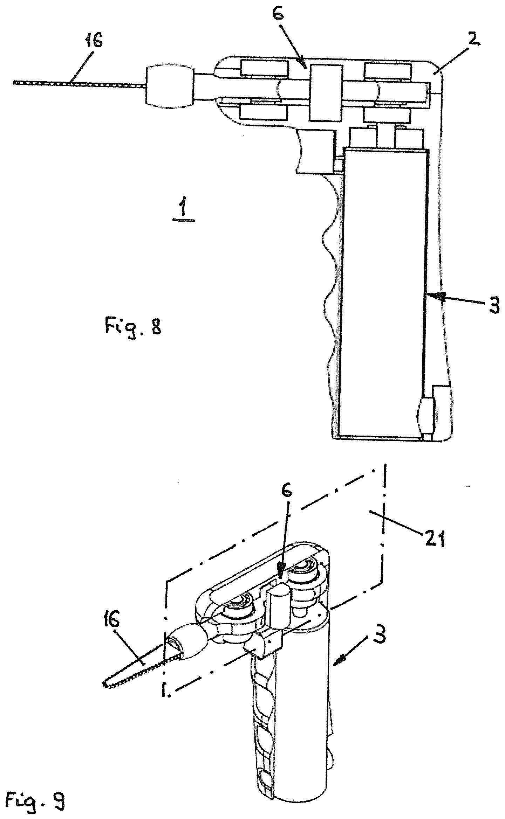

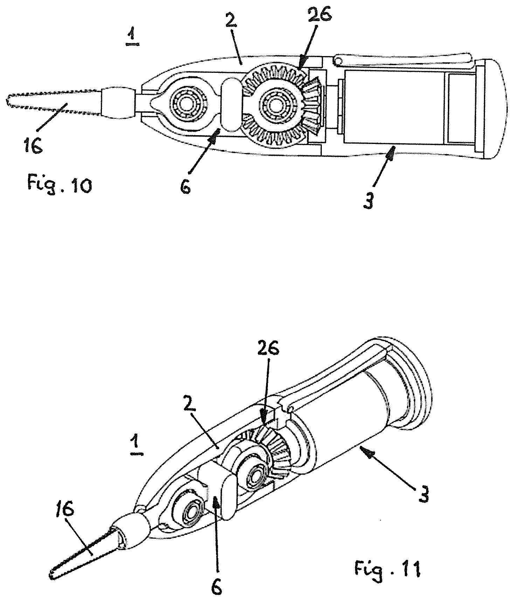

In den Fig. 1 und 2 ist eine erste Ausführungsform der Umlenkeinrichtung 6 der erfindungsgemässen chirurgischen Sägevorrichtung 1 dargestellt. Ferner sind zur besseren Übersicht schematisch Teile des Gehäuses 2 und der Antriebseinheit 3 dargestellt. Die Antriebseinheit 3 ist in das Gehäuse 2 integriert (Fig. 8 und 9) oder am Gehäuse 2 befestigt (Fig. 10 und 11) und hat eine Antriebsachse 5 mit einer Zentralachse 4, wobei mittels der Antriebseinheit 3 eine Rotationsbewegung der Antriebsachse 5 um die Zentralachse 4 erzeugbar ist.

Die in den Fig. 1 und 2 dargestellte Umlenkeinrichtung 6 umfasst im Wesentlichen einen im Gehäuse 2 bewegbar angeordneten Pleuel 7, eine mit der Antriebsachse 5 und dem Pleuel 7 verbundene Exzenterwelle 11 und eine Führungseinrichtung 17 für den Pleuel 7. Die Exzenterwelle 11 umfasst einen zur Zentralachse 4 der Antriebsachse 5 koaxial angeordneten ersten Abschnitt 12, welcher mit der Antriebsachse 5 verbunden ist, und einen zweiten Abschnitt 13, welcher gegenüber dem ersten Abschnitt 12 exzentrisch angeordnet ist. Dabei sind die Achsen des ersten und zweiten Abschnitts 12, 13 parallel angeordnet. Dabei weist der exzentrische zweite Abschnitt 13 gegenüber dem ersten Abschnitt 12 der Exzenterwelle 11 eine Exzentrizität e auf, welche je nach Anwendung der Sägevorrichtung in einem Bereich zwischen 0,25 mm und 0,5 mm liegt.

Die Exzenterwelle 11 ist im Gehäuse 2 konzentrisch zum ersten Abschnitt 12, welcher koaxial zur Zentralachse 4 der Antriebsachse 5 angeordnet ist, gelagert und kann einen weiteren zum ersten Abschnitt 12 koaxialen Abschnitt enthalten, so dass die Exzenterwelle 11 in einem zweiteiligen Gehäuse 2 an je einem zur Zentralachse 4 der Antriebsachse 5 koaxialen Abschnitt gelagert werden kann.,

Der im Gehäuse 2 bewegbar angeordnete Pleuel 7 umfasst eine Längsachse 8, einen hinteren Abschnitt 9 und einen vorderen Abschnitt 10 mit Mitteln zur Befestigung eines Sägeblatts 16. Im hinteren Abschnitt 9 des Pleuels 7 ist ein Lager 14 für den exzentrischen zweiten Abschnitt 13 der Exzenterwelle 11 angeordnet, welches beispielhaft, und nicht einschränkend, als Wälzlager ausgebildet ist. Alternativ kann das Lager 14 auch als Gleitlager ausgebildet sein. Der vordere Abschnitt 10 des Pleuels 7 umfasst eine Befestigungseinrichtung 15 für das Sägeblatt 16.

Die Führungseinrichtung 17 für den Pleuel 7 dient dazu, die durch den exzentrischen zweiten Abschnitt 13 der Exzenterwelle 11 auf das Lager 14 im hinteren Abschnitt 9 ausgeübte Kreisbewegung in eine kombinierte translatorische Bewegung des Pleuels 7 in zwei zueinander orthogonalen Achsrichtungen zu transformieren.

Die Führungseinrichtung 17 umfasst relativ zum Gehäuse 2 bewegbare erste Mittel 18 zur Begrenzung der Pleuelbewegung und relativ zum Gehäuse 2 bewegbare zweite Mittel 19, welche die Bewegung der ersten Mittel 18 einschränken. Durch die ersten Mittel 18 wird eine zur Längsachse 8 des Pleuels 7 koaxiale Translationsbewegung des Pleuels 7 erzwungen, während durch die zweiten Mittel 19 eine Bewegung der ersten Mittel 18 transversal zur Längsachse 8 des Pleuels 7 erzwungen wird. Der Pleuel 7 bewegt sich somit koaxial zu seiner Längsachse 8 und zugleich in einer zur Längsachse 8 orthogonalen Querrichtung, wobei die Längsachse 8 des Pleuels 7 parallel verschoben wird. Die Kreisbewegung des Lagers 14 im hinteren Abschnitt 9 des Pleuels 7 wird dadurch auf den vorderen Abschnitt 10 und somit auf das Sägeblatt 16 übertragen.

Der Pleuel 7 hat zwei Seitenflächen 20a, 20b, welche sich parallel zu einer Ebene 21 erstrecken, wobei diese Ebene 21 durch die Längsachse 8 des Pleuels 7 und durch die Lagerachse 22 des Lagers 14 für den exzentrischen Abschnitt 13 der Exzenterwelle 11 im hinteren Abschnitt 9 des Pleuels 7 aufgespannt wird. Die ersten Mittel 18 der Führungseinrichtung 17 sind als Linearführung für die parallelen Seitenflächen 20a, 20b des Pleuels 7 ausgebildet und weisen anliegend an jeder der zwei Seitenflächen 20a, 20b des Pleuels 7 je ein oder mehrere Führungselemente 23 auf.

Die ersten Mittel 18 umfassen ein relativ zum Gehäuse 2 transversal bewegbares Bauteil 24, wobei die Führungselemente 23 der Linearführung an diesem Bauteil 24 angeordnet sind. Die Führungselemente 23 der ersten Mittel 18 sind beispielhaft, und nicht einschränkend, als Wälzlager ausgebildet, welche an dem transversal zur Längsachse 8 des Pleuels 7 bewegbaren Bauteil 24 befestigt sind. Die Linearführung wird somit durch die an dem Bauteil 24 befestigten Wälzlager gebildet.

Die zweiten Mittel 19 bilden eine Transversalführung für das Bauteil 24 relativ zur Längsachse 8 des Pleuels 7. Ebenso umfasst die Transversalführung beispielhaft, und nicht einschränkend, Wälzlager 26, welche so im Gehäuse 2 befestigt sind, dass eine zur Längsachse 8 des Pleuels 7 orthogonale Bewegung des Bauteils 24 ermöglicht wird.

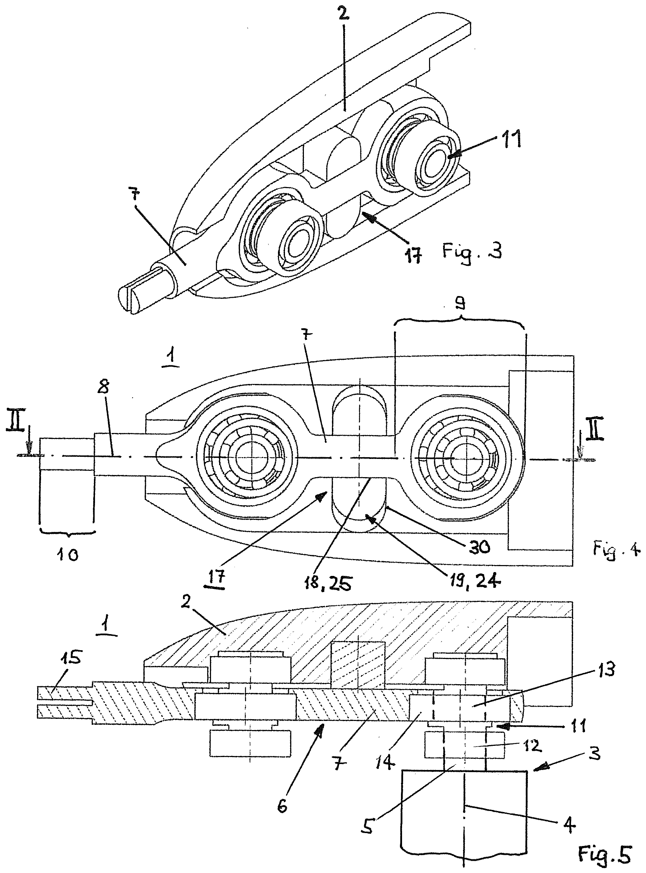

Die in den Fig. 3 - 5 dargestellte Ausführungsform der chirurgischen Sägevorrichtung 1 unterscheidet sich von der in den Fig. 1 und 2 dargestellten Ausführungsform nur darin, dass der Pleuel 7 in einer Nut 25 in dem Bauteil 24 geführt wird, das transversal zur Längsachse 8 des Pleuels 7 bewegbare Bauteil 24 gleitbar in einer weiteren Nut 30 geführt wird, welche orthogonal zur Längsachse 8 des Pleuels 7 im Gehäuse 2 angeordnet ist, und dass der Pleuel 7 in einem zwischen dem vorderen und dem hinteren Abschnitt 9, 10 liegenden Abschnitt an einer passiven zweiten Exzenterwelle 11' gelagert ist. Diese passive zweite Exzenterwelle 11' wird nicht durch die Antriebseinheit 3 angetrieben.

Eine Gesamtdarstellung der Ausführungsform der chirurgischen Sägevorrichtung 1 gemäss den Fig. 3 - 5 findet sich in den Fig. 8 und 9. Das Gehäuse 2 ist zweiteilig ausgebildet, wobei das die Antriebseinheit 3 enthaltende Gehäuseteil als Handgriff ausgebildet ist und sich in Richtung der Zentralachse 4 der Antriebsachse 5 erstreckt. Die Antriebseinheit 3 kann beispielsweise, und nicht einschränkend, einen Elektromotor und Bedienelemente umfassen.

Das Sägeblatt 16 ist longitudinal ausgebildet und kann je nach Anwendung der Sägevorrichtung 1 eine Länge zwischen 15 mm und 100 mm aufweisen. Ferner kann das Sägeblatt 16 in seiner Längsrichtung parallele Seiten aufweisen oder sich gegen das freie Ende des Sägeblatts 16 verjüngen. Die Sägezähne sind an beiden Längsseiten und am freien Ende des Sägeblatts angeordnet, wobei die schneidenden Seiten der Sägezähne analog zu einer Kettensäge auf dem gezahnten Umfangsabschnitt des Sägeblatts 16 in die gleiche Richtung zeigen.

Eine weitere Gesamtdarstellung einer weiteren Ausführungsform der chirurgischen Sägevorrichtung 1 findet sich in den Fig. 10 und 11. Die in den Fig. 10 und 11 dargestellte Ausführungsform unterscheidet sich von der in den Fig. 8 und 9 dargestellten Ausführungsform nur darin, dass die Antriebseinheit 3 so angeordnet ist, dass die Zentralachse 4 der Antriebsachse 5 in der durch die in Längs- und Transversalrichtung bewegte Längsachse 8 des Pleuels 7 aufgespannten Ebene liegt und sich je nach Lage des Pleuels 7 koaxial oder parallel zur Längsachse 8 des Pleuels 7 erstreckt, und dass zwischen der Antriebsachse 5 und der Exzenterwelle 11 eine Zahnradgetriebe 26 angeordnet ist, mittels welchem die Rotationsbewegung der Antriebsachse 5 in die Rotationsbewegung der um 90° abgewinkelten Exzenterwelle 11 umgelenkt wird.

Die in den Fig. 8 - 11 vorgestellten Bauformen (Inline- und Pistolengriff) sind für verschiedenartige Applikationen vorgesehen, um dem Operateur eine ideale Handhabung, Körperhaltung, Patientenlagerung, Sichtfeld, Kraftaufwand etc. zu ermöglichen.

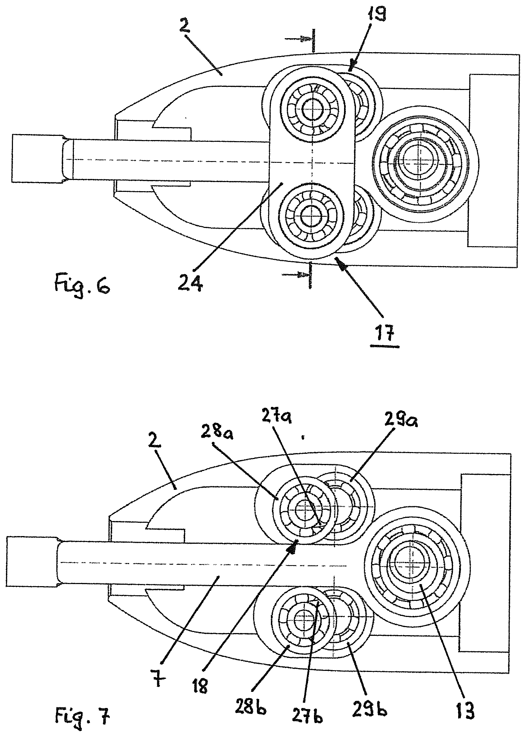

Die in den Fig. 6 und 7 dargestellte Ausführungsform der chirurgischen Sägevorrichtung 1 unterscheidet sich von der in den Fig. 1 und 2 dargestellten Ausführungsform nur darin, dass das Bauteil 24 der ersten Mittel 18 der Führungseinrichtung 17 durch zwei drehbar mit dem Gehäuse 2 verbundene Hebel 27a, 27b schwenkbar am Gehäuse 2 gelagert ist. Das Bauteil 24, die zwei mittels ersten Gelenken 28a, 28b am Bauteil 24 drehbar gelagerten Hebel 27a, 27b und der Gehäuseabschnitt zwischen den beiden am Gehäuse 2 angeordneten zweiten Gelenken 29a, 29b für die Hebel 27a, 27b bilden ein Parallelogramm, dessen Seiten um die ersten, respektive zweiten Gelenke 28a, 28b, 29a, 29b gegeneinander drehbar sind. Die ersten und zweiten Gelenke 28a, 28b, 29a, 29b umfassen beispielhaft, und nicht einschränkend, Wälzlager. Alternativ können anstelle der Wälzlager auch Gleitlager angeordnet sein.

Obwohl wie oben beschrieben verschiedene Ausführungsformen der vorliegenden Erfindung vorliegen, sind diese so zu verstehen, dass die verschiedenen Merkmale sowohl einzeln als auch in jeder beliebigen Kombination verwendet werden können.

Diese Erfindung ist daher nicht einfach auf die oben erwähnten, besonders bevorzugten Ausführungsformen beschränkt.

## Patentansprüche

1. Chirurgische Sägevorrichtung (1) umfassend:
A) ein Gehäuse (2);
B) eine Antriebseinheit (3) mit einer eine Zentralachse (4) aufweisenden Antriebsachse (5), wobei mittels der Antriebseinheit (3) eine Rotationsbewegung der Antriebsachse (5) um die Zentralachse (4) erzeugbar ist;
C) eine Umlenkeinrichtung (6) umfassend:
C1) einen im Gehäuse (2) bewegbar angeordneten Pleuel (7) mit einer Längsachse (8), einem hinteren Abschnitt (9) und einem vorderen Abschnitt (10);
C2) eine Exzenterwelle (11) mit mindestens einem zur Zentralachse (4) der Antriebsachse (5) koaxial angeordneten ersten Abschnitt (12), welcher mit der Antriebsachse (5) verbunden ist, und einem gegenüber dem ersten Abschnitt (12) exzentrischen zweiten Abschnitt (13), wobei der hintere Abschnitt (9) des Pleuels (7) ein Lager (14) für den exzentrischen zweiten Abschnitt (13) der Exzenterwelle (11) und der vordere Abschnitt (10) des Pleuels (7) eine Befestigungseinrichtung (15) für ein Sägeblatt (16) umfasst;
C3) eine Führungseinrichtung (17) für den Pleuel (7),
**dadurch gekennzeichnet, dass**
D) die Führungseinrichtung (17) relativ zum Gehäuse (2) bewegbare erste Mittel (18) zur Begrenzung der Pleuelbewegung umfasst, welche eine zur Längsachse (8) des Pleuels (7) koaxiale Translationsbewegung des Pleuels (7) erzwingen, und
E) die Führungseinrichtung (17) relativ zum Gehäuse (2) bewegbare zweite Mittel (19) zur Begrenzung der Bewegung der ersten Mittel (18) umfasst, welche eine zur Längsachse (8) des Pleuels (7) transversale Bewegung der ersten Mittel (18) erzwinger, wobei sich der Pleuel (7) koaxial zu seiner Längsachse (8) und zugleich in einer zur Längsachse (8) orthogonalen Querrichtung bewegt, wobei die Längsachse (8) des Pleuels (7) parallel verschoben wird.

2. Chirurgische Sägevorrichtung nach Anspruch 1, **dadurch gekennzeichnet, dass** der Pleuel (7) zwei Seitenflächen (20a, 20b) umfasst, welche sich parallel zu einer Ebene (21) erstrecken, wobei die Ebene (21) durch die Längsachse (8) des Pleuels (7) und durch eine Lagerachse (22) des Lagers (14) für den exzentrischen Abschnitt (13) der Exzenterwelle (11) im hinteren Abschnitt (9) des Pleuels (7) aufgespannt wird.

3. Chirurgische Sägevorrichtung nach Anspruch 2, **dadurch gekennzeichnet, dass** die ersten Mittel (18) der Führungseinrichtung (17) eine Linearführung für die parallelen Seitenflächen (20a, 20b) des Pleuels (7) bilden.

4. Chirurgische Sägevorrichtung nach Anspruch 3, **dadurch gekennzeichnet, dass** die Linearführung an jeder der zwei Seitenflächen (20a, 20b) des Pleuels (7) ein oder mehrere Führungselemente (23) aufweist.

5. Chirurgische Sägevorrichtung nach Anspruch 3 oder 4, **dadurch gekennzeichnet, dass** die ersten Mittel (18) ein relativ zum Gehäuse (2) transversal bewegbares Bauteil (24) umfassen, wobei die Führungselemente (23) der Linearführung an oder in diesem Bauteil (24) angeordnet sind.

6. Chirurgische Sägevorrichtung nach Anspruch 3 oder 4, **dadurch gekennzeichnet, dass** die zweiten Mittel (19) relativ zur Längsachse (8) des Pleuels (7) eine Transversalführung für das Bauteil (24) bilden.

7. Chirurgische Sägevorrichtung nach einem der Ansprüche 1 bis 6, **dadurch gekennzeichnet, dass** der exzentrische zweite Abschnitt (13) gegenüber dem ersten Abschnitt (12) der Exzenterwelle (11) eine Exzentrizität e in einem Bereich zwischen 0,25 mm und 0,5 mm aufweist.

8. Chirurgische Sägevorrichtung nach einem der Ansprüche 1 bis 7, **dadurch gekennzeichnet, dass** die Antriebsachse (5) der Antriebseinheit (3) eine Drehzahl von mindestens 9'000 Umdrehungen/min., vorzugsweise von mindestens 10'000 Umdrehungen/min. aufweist.

9. Chirurgische Sägevorrichtung nach einem der Ansprüche 1 bis 8, **dadurch gekennzeichnet, dass** die Antriebsachse (5) der Antriebseinheit (3) eine Drehzahl von höchstens 22'000 Umdrehungen/min., vorzugsweise von höchstens 20'000 Umdrehungen/min. aufweist.

10. Chirurgische Sägevorrichtung nach einem der Ansprüche 3 bis 9, **dadurch gekennzeichnet, dass** die Linearführung und/oder die Transversalführung zwei oder mehr Wälzlager (26) umfasst.

11. Chirurgische Sägevorrichtung nach einem der Ansprüche 3 bis 10, **dadurch gekennzeichnet, dass** die chirurgische Sägevorrichtung (1) zusätzlich ein Sägeblatt (16) umfasst.

## Claims

1. Surgical saw apparatus (1) comprising:
A) a housing (2);
B) a drive unit (3) with a drive axle (5) comprising a central axis (4), wherein a rotation movement of the drive axle (5) around the central axis (4) can be generated by members of the drive unit (3);
C) a deflection device (6) comprising:
C1) a connecting rod (7) that is arranged movably in the housing (2), with a longitudinal axis (8), with a rear section (9) and with a front section (10);
C2) an eccentric shaft (11) with at least one first section (12), which is arranged coaxially with the central axis (4) of the drive axle (5) and is connected to the drive axle (5), and with a second section (13) which is eccentric relative to the first section (12), wherein the rear section (9) of the connecting rod (7) comprises a bearing (14) for the eccentric second section (13) of the eccentric shaft (11), and the front section (10) of the connecting rod (7) comprises a fastening device (15) for a saw blade (16),
C3) a guiding device (17) for the connecting rod (7),
**characterized in that**
D) the guiding device (17) comprises first members (18) for limiting a movement of the connecting rod (7), which are movable relative to the housing (2) and which force a translational movement of the connecting rod (7) that is coaxial with the longitudinal axis (8) of the connecting rod (7), and
E) the guiding device (17) comprises second members (19) for limiting the movement of the first members (18), which are movable relative to the housing (2) and which force a movement of the first members (18) that is transverse with respect to the longitudinal axis (8) of the connecting rod (7),
wherein the connecting rod (7) moves coaxially with its longitudinal axis (8) and at the same time in a transverse direction that is orthogonal with respect to the longitudinal axis (8), wherein the longitudinal axis (8) of the connecting rod (7) is displaced in parallel.

2. Surgical saw apparatus according to claim 1,
**characterized in that**
the connecting rod (7) comprises two side surfaces (20a, 20b) extending parallel to a plane (21), said plane (21) being spanned by the longitudinal axis (8) of the connecting rod (7) and by a bearing axis (22) of the bearing (14) for the eccentric section (13) of the eccentric shaft (11) in the rear section (9) of the connecting rod (7).

3. Surgical saw apparatus according to claim 2,
**characterized in that**
the first members (18) of the guiding device (17) form a linear guidance for the parallel side surfaces (20a, 20b) of the connecting rod (7).

4. Surgical saw apparatus according to claim 3,
**characterized in that**
the linear guidance comprises one guiding element (23) or a plurality of guiding elements (23) on each of the two side surfaces (20a, 20b) of the connecting rod (7).

5. Surgical saw apparatus according to claim 3 or 4,
**characterized in that**
the first members (18) comprise a structural component (24), which is movable transversally to the housing (2), wherein the guiding elements (23) of the linear guidance are arranged on or in said structural component (24).

6. Surgical saw apparatus according to claim 3 or 4,
**characterized in that**
the second members (19) form a transversal guidance for the structural component (24) relative to the longitudinal axis (8) of the connecting rod (7).

7. Surgical saw apparatus according to one of claims 1 to 6,
**characterized in that**
the eccentric second section (13) has, relative to the first section (12) of the eccentric shaft (11), an eccentricity e in a range between 0.25 mm and 0.5 mm.

8. Surgical saw apparatus according to one of claims 1 to 7,
**characterized in that**
the drive axle (5) of the drive unit (3) has a rotation speed of at least 9,000 rpm, preferably at least 10,000 rpm.

9. Surgical saw apparatus according to one of claims 1 to 8,
**characterized in that**
the drive axle (5) of the drive unit (3) has a rotation speed of maximally 22,000 rpm, preferably maximally 20,000 rpm.

10. Surgical saw apparatus according to one of claims 3 to 9,
**characterized in that**
the linear guidance and/or the transversal guidance comprise/comprises two or more roller bearings (26).

11. Surgical saw apparatus according to one of claims 3 to 10,
**characterized in that**
the surgical saw apparatus (1) additionally comprises a saw blade (16).

## Revendications

1. Dispositif de scie chirurgicale (1), comprenant :
A) un boîtier (2) ;
B) une unité d'entraînement (3) avec un arbre d'entraînement (5) ayant un axe central (4), où un mouvement rotatif de l'arbre d'entraînement (5) autour de l'axe central (4) peut être généré au moyen de l'unité d'entraînement (3) ;
C) un dispositif déflecteur (6) comprenant :
C1) une bielle (7) disposée de manière mobile dans le boîtier (2), avec un axe longitudinal (8), une section arrière (9) et une section avant (10) ;
C2) un arbre excentrique (11) avec au moins une première section (12) disposée coaxialement avec l'axe central (4) de l'arbre d'entraînement (5) et reliée à l'arbre d'entraînement (5), et avec une deuxième section (13) excentrique par rapport à la première section (12), la section arrière (9) de la bielle (7) comprenant un palier (14) pour la deuxième section excentrique (13) de l'arbre excentrique (11) et la section avant (10) de la bielle (7) comprenant un dispositif de fixement (15) pour une lame de scie (16),
C3) un dispositif de guidage (17) pour la bielle (7),
**caractérisé en ce que**
D) le dispositif de guidage (17) comprend des premiers moyens (18) mobiles par rapport au boîtier (2) pour limiter le mouvement de la bielle (7), lesquels forcent un mouvement translationnel de la bielle (7) coaxial avec l'axe longitudinal (8) de la bielle (7), et
E) le dispositif de guidage (17) comprend des deuxièmes moyens (19) mobiles par rapport au boîtier (2) pour limiter le mouvement des premiers moyens (18), lesquels forcent un mouvement des premiers moyens (18) transversale à l'axe longitudinal (8) de la bielle (7),
où la bielle (7) se meut coaxialement avec son axe longitudinal (8) et en même temps dans une direction transversale qui est orthogonale à l'axe longitudinal (8), l'axe longitudinal (8) de la bielle (7) étant déplacé en parallèle.

2. Dispositif de scie chirurgicale selon la revendication 1,
**caractérisé en ce que**
la bielle (7) comprend deux surfaces latérales (20a, 20b) qui s'étendent en parallèle à un plan (21), ledit plan (21) étant tendu par l'axe longitudinal (8) de la bielle (7) et par un axe-palier (22) du palier (14) pour la section excentrique (13) de l'arbre excentrique (11) dans la section arrière (9) de la bielle (7).

3. Dispositif de scie chirurgicale selon la revendication 2,
**caractérisé en ce que**
les premier moyens (18) du dispositif de guidage (17) forment un guidage linéaire pour les surfaces latérales (20a, 20b) de la bielle (7).

4. Dispositif de scie chirurgicale selon la revendication 3,
**caractérisé en ce que**
le guidage linéaire comprend un ou plusieurs élément/s de guidage (23) sur chacune des deux surfaces latérales (20a, 20b) de la bielle (7).

5. Dispositif de scie chirurgicale selon la revendication 3 ou 4,
**caractérisé en ce que**
les premiers moyens (18) comprennent un composant qui est mouvable transversalement par rapport au boîtier (2), les éléments de guidage (23) du guidage linéaire étant disposés sur ou dans ce composant (24).

6. Dispositif de scie chirurgicale selon la revendication 3 ou 4,
**caractérisé en ce que**
les deuxièmes moyens (19) forment, par rapport à l'axe longitudinal (8) de la bielle (7), un guidage transversal pour le composant (24).

7. Dispositif de scie chirurgicale selon l'une des revendications 1 à 6,
**caractérisé en ce que**
la deuxième section excentrique (13) présente par rapport à la première section (12) de l'arbre excentrique (11) une excentricité e dans une plage entre 0,25 mm et 0,5 mm.

8. Dispositif de scie chirurgicale selon l'une des revendications 1 à 7,
**caractérisé en ce que**
l'axe d'entraînement (5) de l'unité d'entraînement (3) présente une vitesse rotative d'au moins 9 000 tours/min, de préférence d'au moins 10 000 tours/min.

9. Dispositif de scie chirurgicale selon l'une des revendications 1 à 8,
**caractérisé en ce que**
l'axe d'entraînement (5) de l'unité d'entraînement (3) présente une vitesse rotative d'au plus 22 000 tours/min, de préférence d'au plus 20 000 tours/min.

10. Dispositif de scie chirurgicale selon l'une des revendications 3 à 9,
**caractérisé en ce que**
le guidage linéaire et/ou le guidage transversal comprend deux paliers à roulement (26) ou plus.

11. Dispositif de scie chirurgicale selon l'une des revendications 3 à 10,
**caractérisé en ce que**
le dispositif de scie chirurgicale (1) comprend en outre une lame de scie (16).
